Europäisches Patentamt

European Patent Office

Office européen des brevets

(11) Numéro de publication: **0 462 015 B1**

## (12) FASCICULE DE BREVET EUROPEEN

(45) Date de publication du fascicule du brevet: **22.11.95**   (51) Int. Cl.⁶: **A61K  9/52**

(21) Numéro de dépôt: **91401581.3**

(22) Date de dépôt: **14.06.91**

Le dossier contient des informations techniques
présentées postérieurement au dépôt de la
demande et ne figurant pas dans le présent
fascicule.

(54) **Procédé d'enrobage par un polymère pH sensible de principes actifs.**

(30) Priorité: **15.06.90 FR 9007584**

(43) Date de publication de la demande:
**18.12.91 Bulletin  91/51**

(45) Mention de la délivrance du brevet:
**22.11.95 Bulletin  95/47**

(84) Etats contractants désignés:
**AT BE CH DE DK ES FR GB GR IT LI LU NL SE**

(56) Documents cités:
**EP-A- 0 058 765        EP-A- 0 077 264
EP-A- 0 260 186        EP-A- 0 268 533
FR-A- 2 145 642        FR-A- 2 401 621
US-A- 4 853 461**

(73) Titulaire: **RHONE-POULENC NUTRITION ANI-
MALE
Rue Marcel Lingot
F-03600 Commentry (FR)**

(72) Inventeur: **Prud'homme, Christian
19 rue Commandant Faurax
F-69006 Lyon (FR)**
Inventeur: **Ardaillon, Pierre
10 Impasse Beau-Vallon
F-69800 Saint-Priest (FR)**

(74) Mandataire: **Le Pennec, Magali et al
RHONE-POULENC RORER SA,
Direction des Brevets,
20 Avenue Raymond Aron
F-92165 Antony Cédex (FR)**

## Description

La présente invention concerne un procédé de préparation d'un polymère pH sensible ainsi qu'un procédé d'enrobage à partir de ce polymère de principes actifs. Elle concerne plus particulièrement un procédé de préparation du polymère pH sensible en émulsion et son utilisation pour l'enrobage, de principes actifs médicamenteux et/ou alimentaires.

Il est connu selon les brevets publiés sous les numéros FR 2145642 et EP 58765 d'enrober des médicaments à usage humain avec un polymère en émulsion aqueuse. Ces polymères sont pour la plupart stables en milieu stomacal et libérables dans l'intestin, ils ne peuvent donc pas convenir à une utilisation pour traiter les ruminants. En plus en milieu neutre ou alcalin ils libèrent le principe actif enrobé en quelques secondes.

Il est connu par exemple selon le brevet US 4 593 082 de préparer un copolymère à base de vinylpyridine et de styrène par polymérisation en émulsion des monomères en présence d'un agent surfactant choisi parmi les sels alcalins d'acides gras et d'un initiateur à un pH fixé entre 10 et 14. Dans tous les cas le polymère est récupéré par filtration, lavé puis séché.

Il est également connu depuis longtemps d'enrober des principes actifs par des polymères PH sensibles, éventuellement mélangés avec une substance hydrophobe, telle que l'acide stéarique, et/ou un polymère non hydrosoluble tel que l'éthylcellulose. Ces enrobages sont particulièrement décrits en ce qui concerne l'alimentation animale dans les demandes de brevets publiées sous les numéros FR 2 401 621, US 4 832 967, EP 77 264, EP 260 186 ou EP 188 953.

Selon ces demandes on enrobe les particules sphériques de méthionine et/ou de lysine par la technique du lit fluidisé à l'aide d'une solution de polymère PH sensible, préalablement synthétisé et isolé par exemple selon le brevet précédemment décrit que l'on dissout ensuite pour l'étape d'enrobage dans un ou plusieurs solvants organiques tels que les solvants halogénés, les alcools ou les éthers. Cette technique permet une pulvérisation facile et homogène du ou des polymères PH sensibles et des additifs mais présente l'inconvénient du point de vue économique de devoir isoler le polymère et du point de l'écotoxicité de devoir introduire une quantité importante de solvants, environ 20 à 100 g de solvants pour dissoudre 1 g de polymère PH sensible. Il est regrettable de devoir introduire sur des produits destinés à l'alimentation animale des solvants dont la non toxicité n'est pas reconnue.

La présente invention a permis de procéder directement à la synthèse du polymère et son utilisation pour l'enrobage sans étape intermédiaire d'isolement. Ce procédé permet ainsi l'enrobage des principes actifs tels que les acides aminés ou les vitamines en évitant l'isolement du polymère et tout usage, lors de l'étape d'enrobage, de solvants organiques non compatibles avec les milieux biologiques.

En effet la présente invention concerne un procédé d'enrobage, par une composition à base d'un polymère PH sensible, de principes actifs médicamenteux et/ou alimentaires, destinés aux ruminants, peu ou pas dégradables dans le rumen et libérables dans la caillette et/ou l'intestin caractérisé en ce que dans une première étape on polymérise en émulsion aqueuse un monomère ou un mélange de monomères, dont la polymérisation permet la synthèse du polymère pH sensible, en présence d'un agent surfactant, puis dans une deuxième étape sans isolement intermédiaire on dépose sur les dits principes actifs l'émulsion aqueuse du polymère pH sensible après addition éventuelle d'agents supplémentaires d'enrobage. Il est tout à fait étonnant que le procédé puisse être mis en oeuvre, malgré l'hydrosolubilité importante de certains principes actifs à enrober comme notamment la lysine ou ses dérivés. Les principes actifs médicamenteux et/ou alimentaires à enrober se présentent de préférence sous forme de granulés notamment sphériques ou ovoides ayant un diamètre compris entre 0,3 et 3 mm.

L'enrobage à partir de l'émulsion aqueuse de polymérisation permet une économie importante dans la mise en oeuvre du procédé, car elle évite l'opération d'isolement du polymère, l'utilisation de solvant de redissolution du polymère et l'utilisation conséquente d'installations de récupération de solvants, coûteuses du point de vue sécurité et investissement. Elle permet aussi d'augmenter les productivités par rapport aux procédés utilisés dans l'art antérieur.

Les polymères PH sensibles, dont la préparation est réalisée par polymérisation en émulsion, utilisés dans le procédé selon l'invention, sont choisis notamment parmi :
- les polyvinyl acétals d'esters acétylacétiques substitués par des groupes azotés dialkylés tels que le groupe diéthylamino,
- les copolymères du styrène ou de l'acrylonitrile avec les isomères ou les dérivés de la vinylpyridine.

On préfère parmi l'ensemble des polymères cités utiliser les copolymères du styrène avec la vinyl pyridine et tout particulièrement le copolymère à base de styrène et de vinyl-2 pyridine.

La préparation du polymère est réalisée de manière classique par mise en contact du ou des monomères en présence d'un agent surfactant et d'un initiateur de polymérisation. Les agents surfactants sont choisis de préférence parmi les sels alcalins d'acides gras, on préfère tout particulièrement utiliser le sel de sodium de l'acide oléique. L'initiateur de polymérisation est choisi parmi les initiateurs solubles utilisés classiquement dans les procédés en émulsion, on préfère tout particulièrement utiliser le persulfate de sodium. Le pH au cours de la polymérisation est de préférence fixé entre 10 et 14 et tout particulièrement entre 11 et 13.

Les principes actifs médicamenteux pouvant être enrobés par le procédé de l'invention sont choisis notamment parmi les vitamines, les antibiotiques, les composés antiparasitaires, les hormones. Les principes actifs alimentaires sont choisis notamment parmi les acides aminés essentiels supposés limitant tels que la méthionine et/ou la lysine et/ou le tryptophane.

On peut adjoindre à ces principes actifs des charges, éventuellement lamellaires, qui facilitent le délitement dans le tractus digestif. Ces charges sont notamment choisies parmi les charges pH sensibles ou non telles que par exemple: le talc et/ou la silice et/ou les carbonates et/ou les polyphosphates complexes tels que à base de $Na_2O$, $CaO$, $P_2O_5$ et $Al_2O_3$. On peut également adjoindre à ces principes actifs des agents liants choisis parmi les acides ou les esters gras, la cellulose (telle que celle commercialisée, par exemple, sous la dénomination Avicel) ou ses dérivés, notamment l'éthylcellulose, la carboxyméthylcellulose, ainsi que les copolymères basiques. L'ensemble du ou des principes actifs et des additifs forment le coeur du granulé qui est ensuite enrobé par l'émulsion du polymère PH sensible préalablement préparée.

Ces principes actifs médicamenteux ou alimentaires avec éventuellement un additif et enrobés de polymère PH sensible sont particulièrement intéressants pour l'alimentation des ruminants car ils sont peu ou pas dégradés lors du transit dans le rumen et sont libérés dans la caillette et/ou l'intestin des ruminants.

L'enrobage est réalisé par dépôt de l'émulsion aqueuse de polymérisation contenant le polymère pH sensible. L'émulsion d'enrobage peut aussi contenir des additifs tels que ceux mentionnés précédemment, ainsi que des agents antistatiques, des agents plastifiants, des colorants ou des agents d'apétence et des agents émulsifiants complémentaires. On préfère utiliser, comme mélange d'enrobage, une dispersion aqueuse contenant le polymère PH sensible, une substance hydrophobe, contenant éventuellement un polymère non hydrosoluble et contenant un agent émulsifiant.

La substance hydrophobe est choisie de préférence parmi les acides gras contenant 12 à 22 atomes de carbone tels que par exemple l'acide stéarique ou l'acide béhénique.

L'émulsifiant peut être choisi parmi les esters d'acides gras ou les sels d'acides gras, il peut être le même ou différent de l'agent surfactant utilisé lors de la polymérisation. L'émulsifiant peut dans le cas où il s'agit d'un sel d'acide gras être créé "in situ" par salification de l'acide gras au moyen d'une base choisie parmi les hydroxydes alcalins et d'ammonium.

Le polymère non hydrosoluble est avantageusement choisi parmi les ethers et les esters de cellulose non hydrosolubles tels que l'éthylcellulose, l'acétate de cellulose, le propionate de cellulose, l'acétobutyrate de cellulose.

Le mélange d'enrobage est préparé selon un mode de mise en oeuvre préférentiel, par mélange de l'émulsion aqueuse de polymérisation et d'une solution aqueuse contenant une base, de façon à provoquer la formation "in situ" de l'émulsifiant, mélange dans lequel on introduit l'acide gras fondu et les éventuels agents d'enrobage complémentaires. Il est évident que selon une autre façon de procéder on peut introduire dans l'émulsion de polymérisation un complément d'agent émulsifiant puis l'acide gras et les agents complémentaires d'enrobage.

Ainsi l'émulsion d'enrobage contient notamment 1 à 10 % en poids, par rapport à la composition sèche d'enrobage, d'un émulsifiant choisi, comme mentionné précédemment, parmi les esters ou les sels d'acides gras.

D'un point de vue quantitatif, l'émulsion d'enrobage contient de préférence:
- polymère PH sensible 10 à 70 g
- une ou plusieurs substances hydrophobes 30 à 90 g
- un ou plusieurs polymères non hydrosolubles 0 à 20 g pour 150 millilitres à un litre d'eau.

Il est bien évident que ces quantités sont des indications préférentielles qui seront adaptées par l'homme de l'art en fonction de la viscosité et de la stabilité de l'émulsion.

L'émulsion est ensuite déposée sur les granulés à enrober, par exemple selon la technique du lit fluidisé de type WURSTER telle que décrite dans les brevets US 2 799 241 et EP 188 953. On dépose de préférence une couche d'enrobage ayant une épaisseur de 5 à 100 microns et encore plus préférentiellement de 40 à 60 microns.

Les granulés obtenus après enrobage sont utilisés comme compléments alimentaires ou pour le traitement médicamenteux des ruminants.

Leur préparation sera plus complètement décrite à l'aide des exemples suivants.

EP 0 462 015 B1

EXEMPLE 1

Préparation d'une émulsion de copolymère de vinyl-2 pyridine et de styrène

On mélange 150 g de styrène et 350 g de vinyl-2 pyridine fraîchement distillée. Ce mélange est lavé deux fois avec 160 ml d'une solution aqueuse de soude à 5 %, puis deux fois avec 320 ml d'eau déminéralisée. Les monomères ainsi traités sont chargés dans un réacteur de polymérisation purgé à l'azote.

Dans un autre récipient on prépare une solution basique d'oléate de sodium en coulant 15,25 g d'acide oléique dans 736 g d'eau déminéralisée dans laquelle on a préalablement dissous 7,7 g de soude en pastilles. La coulée de l'acide oléique se fait en dix minutes, à température ambiante, sous agitation modérée.

La solution d'oléate ainsi obtenue est ensuite introduite dans le réacteur de polymérisation et mélangée aux monomères. On dilue le mélange en rajoutant 641 g d'eau déminéralisée, puis on commence à agiter à 200 tours par minute pour former une émulsion.

On purge soigneusement le système en y faisant barboter de l'azote, et l'on commence à chauffer. Lorsque la température de l'émulsion est stabilisée à 50°C, on coule en deux minutes, 100 g de solution aqueuse à 5 % de persulfate de sodium. Le chauffage à 50°C et l'agitation sont maintenus pendant cinq heures.

Après polymérisation, l'émulsion est "strippée" à la vapeur d'eau pour éliminer les traces de monomères résiduels. On obtient ainsi une émulsion aqueuse de copolymère, sa concentration en extrait sec est de 20,75 % en poids et son pH est de 13.

Afin de caractériser le polymère formé, on prélève un échantillon de l'émulsion. Par acidification de cet échantillon, on obtient la coagulation du polymère que l'on sépare par filtration sur verre fritté, et que l'on sèche à l'étuve après plusieurs lavages à l'eau déminéralisée.

Le copolymère séché, en poudre, présente les caractéristiques suivantes:
- viscosité inhérente, mesurée à 25°C, sur une solution de 0,5 g de copolymère dans 100 ml de diméthylformamide = 1,33 dl/g
- masse moléculaire déterminées par GPC (Gel Permeation Chromatographie) dans la N méthylpyrrolidone, par rapport à des étalons de polystyrène:
  Mn = 109000 g/mole
  Mw = 610000 g/mole

Préparation de l'émulsion d'enrobage

Dans un récipient de 2 litres, en verre, on charge 420 g d'eau déminéralisée et 1,8 ml de solution aqueuse de soude à 10 % (p/v). Dans ce mélange chauffé à 84 °C et sous agitation modérée, on coule, en une minute, 106 g de l'émulsion de copolymère préparée précédemment. Dans ce mélange maintenue à 80°C, on ajoute 88 g d'acide stéarique commercialisé par UNICHEMA sous la marque PRIFAC 2981, préalablement chargé dans une ampoule de coulée chauffée à 90°C par un cordon électrique chauffant.

La coulée est effectuée en 2 minutes environ, tandis que le mélange est brassé vigoureusement avec un appareil POLYTRON ! tournant à 11 000 tours/minute. Après la fin de la coulée, on maintient l'agitation pendant encore 2 minutes.

On obtient ainsi une émulsion que l'on maintient à une température supérieure à 70°C, sous agitation modérée (agitation magnétique). La teneur en extrait dans cette émulsion est de 20,4 % (p/p).

Enrobage de granulés d'acides aminés

Dans cet exemple, l'émulsion d'enrobage a été utilisée dans la demi heure suivant la fin de sa préparation.

Dans un appareil de spray-coating UNIGLATT! équipé d'un système WURSTER, on charge 500 g de granulés sphériques de 2 mm de diamètre moyen et titrant 57 % de chlorhydrate de lysine et 16 % de méthionine.

L'émulsion, maintenue à 90°C et doucement agitée, est pulvérisée dans le lit fluidisé formé par les granulés, les conditions de pulvérisation étant les suivantes:
- quantité d'émulsion pulvérisée: 320 g environ
- débit de l'air de fluidisation: 130 m$^3$/h
- température de l'air de fluidisation (sortie):37°C

4

- pression de l'air de pulvérisation: 1,5 bars
- température de l'air de pulvérisation: 85 °C
- débit de l'émulsion: 9 ml/mn
- durée de pulvérisation: 41 mn.

Le degré de protection apporté par la pellicule d'enrobage ainsi déposée est évalué selon un test in vitro consistant à agiter une quantité déterminée de granulés enrobés, dans une solution aqueuse tamponnée à PH6 et maintenue à 40 °C. On effectue un dosage de la quantité de chlorhydrate de lysine libérée dans le milieu après 6 heures et 24 heures. On évalue de même la pH sensibilité de l'enrobage par un test in vitro consistant à agiter une quantité déterminée de granulés enrobés, dans une solution aqueuse tamponnée à pH 2 et maintenue à 40 °C. On dose la quantité de chlorhydrate de lysine libérée dans l'eau après 15 mn et 30 mn respectivement. Les résultats sont indiqués dans le tableau I.

Ces résultats mettent en évidence un taux de protection élevé à pH 6, et une vitesse de libération des acides aminés rapide à pH 2.

EXEMPLE 2

En partant de l'émulsion de copolymère basique préparée précédemment, on reproduit l'exemple 1 en modifiant seulement la durée de stockage de l'émulsion d'enrobage. Celle ci au lieu d'être utilisée presque immédiatement, est d'abord conservée pendant 48 heures à 72 °C avant d'être pulvérisée dans le lit fluidisé de granulés.

Les acides aminés enrobés sont évalués suivant les tests décrits à l'exemple 1. Les résultats indiqués dans le tableau I mettent en évidence des caractéristiques de protection à pH6 et de libération à pH2 tout à fait satisfaisantes pour l'application.

EXEMPLE 3

On part toujours de l'émulsion de copolymère basique préparée dans l'exemple 1.
On reproduit l'exemple 1 en modifiant seulement la teneur en extrait sec de l'émulsion d'enrobage.
On passe de 20,4 % à 30,2 % en utilisant 236 g d'eau déminéralisée au lieu de 420 g, pour la dilution du copolymère basique.
Les résultats de cet essai sont également donnés dans le tableau I.

EXEMPLE 4

On procède comme dans les exemples 1 et 3. On modifie simplement la quantité d'eau déminéralisée utilisée pour diluer l'émulsion de copolymère basique dans la première phase de la préparation de l'émulsion d'enrobage.

Dans cet exemple on a donc utilisé 130 g d'eau, ce qui a conduit à une émulsion finale à 38,3 % d'extrait sec.

TABLEAU I

| Echantillon | Emulsion teneur en extrait sec (% p/p) | Emulsion Durée de stockage (H) avant utilisation | Durée de pulvérisation (mn) | Taux d'enrobant sur les granulés (%, poids) | Test in vitro:PH6/40°C % de lysine libérée après 6 H | Test in vitro:PH6/40°C % de lysine libérée après 24 H | Test in vitro:PH2/40°C % de lysine libérée après 15 mn | Test in vitro:PH2/40°C % de lysine libérée après 30 mn |
|---|---|---|---|---|---|---|---|---|
| Exemple 1 | 20,4 | < 0,5 | 41 | 11,6 | 2,4 | 5,0 | 92 | 100 |
| Exemple 2 | 20,7 | 48 | 41 | 10,2 | 0 | 4,5 | 90 | 100 |
| Exemple 3 | 30,2 | < 0,5 | 27 | 10,2 | 1,7 | 3,5 | 90 | 100 |
| Exemple 4 | 38,3 | < 0,5 | 21 | 9,0 | 2,8 | 7,7 | 93 | 100 |

**Revendications**

1. Procédé d'enrobage par une composition à base d'un polymère pH sensible de principes actifs médicamenteux et/ou alimentaires destinés aux ruminants peu ou pas dégradables dans le rumen et

6

libérables dans la caillette et/ou l'intestin caractérisé en ce dans une première étape on polymérise en émulsion aqueuse un monomère ou un mélange de monomères, dont la polymérisation permet la synthèse du polymère pH sensible, en présence d'un agent surfactant, puis dans une deuxième étape sans isolement intermédiaire, on dépose sur lesdits principes actifs, l'émulsion aqueuse du polymère pH sensible après addition éventuelle d'agents supplémentaires d'enrobage.

2. Procédé selon la revendication 1 caractérisé en ce que dans la première étape l'agent surfactant est l'oléate de sodium et en ce que dans la deuxième étape on ajoute comme agent supplémentaire d'enrobage une substance hydrophobe et/ou un polymère non hydrosoluble.

3. Procédé selon la revendication 1 caractérisé en ce que le principe actif est un acide aminé choisi parmi la méthionine et/ou la lysine ou un de ses dérivés.

4. Procédé selon la revendication 2 caractérisé en ce que la substance hydrophobe est choisie parmi les acides gras contenant 12 à 22 atomes de carbone et de préférence est l'acide stéarique.

5. Procédé selon la revendication 2 caractérisé en ce que le polymère non hydrosoluble est choisi parmi les éthers et les esters de cellulose non hydrosolubles.

6. Procédé selon la revendication 2 caractérisé en ce que on ajoute dans la deuxième étape un agent émulsifiant, choisi parmi les sels et les esters d'acides gras.

7. Procédé selon la revendication 1 caractérisé en ce qu'on ajoute au principe actif une charge choisie parmi les charges telles que : le talc et/ou la silice et/ou les carbonates et/ou les polyphosphates complexes tels que à base de $Na_2O$, $CaO$, $P_2O_5$ et $Al_2O_3$.

8. Procédé selon l'une quelconque des revendications précédentes caractérisé en ce que dans une première étape on polymérise en émulsion un mélange de vinylpyridine et de styrène en présence d'oléate de sodium en ce que dans une deuxième étape on ajoute de l'acide stéarique, une solution aqueuse d'hydroxyde de sodium et que l'on pulvérise cette émulsion sur des granulés de méthionine et/ou de lysine.

9. Procédé selon l'une quelconque des revendications précédentes caractérisé en ce que l'émulsion d'enrobage contient en plus un ou plusieurs additifs choisis parmi les charges telles que définies à la revendication 7, des agents antistatiques, des agents plastifiants, des colorants et des agents d'apétence.

10. Procédé selon l'une quelconque des revendications précédentes caractérisé en ce que l'émulsion d'enrobage est composée de:
   - polymère pH sensible 10 à 70 g
   - substance hydrophobe 30 à 90 g
   - polymère non hydrosoluble 0 à 20 g
   pour 150 millilitres à un litre d'eau.

## Claims

1. Process for coating, using a pH-sensitive-polymer-based composition, medicinal and/or dietary active principles intended for ruminants and which are poorly or not degradable in the rumen and can be released inside the abomasum and/or the intestine, characterised in that, in a first stage, a monomer or a mixture of monomers, whose polymerisation allows the synthesis of the pH-sensitive polymer, is polymerised in aqueous emulsion in the presence of a surfactant and then, in a second stage, the aqueous emulsion of the pH-sensitive polymer, after the optional addition of additional coating agents, is deposited on the said active principles without intermediate isolation.

2. Process according to claim 1, characterised in that, in the first stage, the surfactant is sodium oleate and in that, in the second stage, a hydrophobic substance and/or a water-insoluble polymer is added as additional coating agent.

3. Process according to claim 1, characterised in that the active principle is an amino acid chosen from methionine and/or lysine or one of its derivatives.

4. Process according to claim 2, characterised in that the hydrophobic substance is chosen from fatty acids containing 12 to 22 carbon atoms and is preferably stearic acid.

5. Process according to claim 2, characterised in that the water-insoluble polymer is chosen from water-insoluble cellulose ethers and esters.

6. Process according to claim 2, characterised in that, in the second stage, an emulsifying agent, chosen from fatty acid salts and esters, is added.

7. Process according to claim 1, characterised in that there is added to the active principle a filler chosen from fillers such as: talc and/or silica and/or carbonates and/or complex polyphosphates such as those based on $Na_2O$, $CaO$, $P_2O_5$ and $Al_2O_3$.

8. Process according to any one of the preceding claims, characterised in that, in a first stage, a mixture of vinylpyridine and styrene is polymerised in emulsion in the presence of sodium oleate, in that, in a second stage, stearic acid and an aqueous sodium hydroxide solution are added, and in that this emulsion is sprayed on methionine and/or lysine granules.

9. Process according to any one of the preceding claims, characterised in that the coating emulsion furthermore contains one or more additives chosen from the fillers defined in claim 7, antistatic agents, plasticizers, colourings and appetising agents.

10. Process according to any one of the preceding claims, characterised in that the coating emulsion is composed of:
   - 10 to 70 g of pH-sensitive polymer
   - 30 to 90 g of hydrophobic substance
   - 0 to 20 g of water-insoluble polymer
per 150 millilitres to one litre of water.

**Patentansprüche**

1. Verfahren zur Umhüllung von Arzneimittel- und/oder Nahrungs- bzw. Futtermittelwirkstoffen, die für Wiederkäuer bestimmt sind, und wenig oder gar nicht im Pansen abbaubar sind, und im Labmagen und/oder Darmtrakt freisetzbar sind, mit einer Zusammensetzung auf Basis eines pH-empfindlichen Polymeren, dadurch gekennzeichnet, daß man in einer ersten Stufe ein Monomeres oder ein Monomerengemisch, dessen Polymerisation die Synthese des pH-empfindlichen Polymeren erlaubt, in wäßriger Emulsion in Gegenwart eines oberflächenaktiven Mittels polymerisiert, dann in einer zweiten Stufe ohne Zwischenisolierung auf den genannten Wirkstoffen die wäßrige Emulsion des pH-empfindlichen Polymeren nach eventueller Zugabe von ergänzenden Umhüllungsmitteln ablagert.

2. Verfahren gemäß Anspruch 1, dadurch gekennzeichnet, daß in der ersten Stufe das oberflächenaktive Mittel Natriumoleat ist, und daß man in der zweiten Stufe als ergänzendes Umhüllungsmittel eine hydrophobe Substanz und/oder ein nichtwasserlösliches Polymeres zusetzt.

3. Verfahren gemäß Anspruch 1, dadurch gekennzeichnet, daß der Wirkstoff eine Aminosäure ist, ausgewählt unter Methionin und/oder Lysin und/oder seinen Derivaten.

4. Verfahren gemäß Anspruch 2, dadurch gekennzeichnet, daß die hydrophobe Substanz ausgewählt ist unter den Fettsäuren mit 12 bis 22 Kohlenstoffatomen und dies vorzugsweise die Stearinsäure ist.

5. Verfahren gemäß Anspruch 2, dadurch gekennzeichnet, daß das nichtwasserlösliche Polymere ausgewählt ist unter den nichtwasserlöslichen Celluloseethern und -estern.

6. Verfahren gemäß Anspruch 2, dadurch gekennzeichnet, daß man in der zweiten Stufe ein Emulgiermittel, ausgewählt unter den Fettsäuresalzen und -estern zusetzt.

EP 0 462 015 B1

**7.** Verfahren gemäß Anspruch 1, dadurch gekennzeichnet, daß man dem Wirkstoff einen Füllstoff zusetzt, ausgewählt unter Füllstoffen wie Talk und/oder Siliziumdioxid und/oder Carbonaten und/oder komplexen Polyphosphaten wie auf Basis von $Na_2O$, $CaO$, $P_2O_5$ und $Al_2O_3$.

**8.** Verfahren gemäß einem der vorhergehenden Ansprüche, dadurch gekennzeichnet, daß man in einer ersten Stufe ein Gemisch von Vinylpyridin und Styrol in Gegenwart von Natriumoleat in Emulsion polymerisiert, und daß man in einer zweiten Stufe Stearinsäure, eine wäßrige Natriumhydroxidlösung zusetzt, und daß man diese Emulsion auf Granulaten von Methionin und/oder Lysin zerstäubt.

**9.** Verfahren gemäß einem der vorhergehenden Ansprüche, dadurch gekennzeichnet, daß die Umhüllungsemulsion außerdem einen oder mehrere Zusatz/Zusätze enthält, ausgewählt unter den Füllstoffen, wie sie in Anspruch 7 definiert sind, antistatischen Mitteln, Plastifiziermitteln bzw. Weichmachern, Farbstoffen und appetitanregenden Mitteln.

**10.** Verfahren gemäß einem der vorhergehenden Ansprüche, dadurch gekennzeichnet, daß die Umhüllungsemulsion zusammengesetzt ist aus:
- 10 bis 70 g pH-empfindliches Polymeres,
- 30 bis 90 g hydrophobe Substanz,
- 0 bis 20 g nichtwasserlösliches Polymeres,

für 150 ml auf 1 Liter Wasser.